(19) Europäisches Patentamt

European Patent Office

Office européen des brevets

(11) Veröffentlichungsnummer: **0 070 522**
**B1**

(12) # EUROPÄISCHE PATENTSCHRIFT

(45) Veröffentlichungstag der Patentschrift:
**29.10.86**

(51) Int. Cl.⁴: **C 12 N 15/00** // C12R1/61

(21) Anmeldenummer: **82106361.7**

(22) Anmeldetag: **15.07.82**

(54) **Plasmid p SVH 1 und seine Verwendung.**

(30) Priorität: **20.07.81 DE 3128669**

(43) Veröffentlichungstag der Anmeldung:
**26.01.83 Patentblatt 83/4**

(45) Bekanntmachung des Hinweises auf die Patenterteilung:
**29.10.86 Patentblatt 86/44**

(84) Benannte Vertragsstaaten:
**AT BE CH DE FR GB IT LI NL SE**

(56) Entgegenhaltungen:
**PLASMID, Band 2, 9. April 1979, (ACADEMIC PRESS Inc.), USA, V.S. MALIK et al.: "Restriction enzyme map for streptomycete plasmid pUC3"**
**JOURNAL OF ANTIBIOTICS, Band 33, Nr. 1, Januar 1980, JP, M. OKANISHI et al.: "Isolation and characterization of plasmid DNAs in actinomycetes"**
**CHEMICAL ABSTRACTS, Band 92, Nr. 9, 3. März 1980, Seite 327, Nr. 72493k, Columbus, Ohio, USA, T. HAYAKAWA et al.: "A linear plasmid-like DNA in streptomyces species producing lankacidin group antibiotics"**
**CHEMICAL ABSTRACTS, Band 84, Nr. 3, 19. Januar 1976, Seite 233, Nr. 14569p, Columbus, Ohio, USA, H. AKAGAWA: "Plasmid involved in chloroamphenicol production in streptomyces venezuelae. Evidence from genetic mapping"**

(73) Patentinhaber: **HOECHST AKTIENGESELLSCHAFT, Postfach 80 03 20, D-6230 Frankfurt am Main 80 (DE)**

(72) Erfinder: **Pühler, Alfred, Prof. Dr., Am Waldschlössen 2, D-4800 Bielefeld (DE)**
Erfinder: **Wohlleben, Wolfgang, Dr., Heidelberger Weg 7, D-4800 Bielefeld (DE)**
Erfinder: **Leineweber, Michael, Dr., Sachsenring 10, D-6238 Hofheim am Taunus (DE)**

(56) Entgegenhaltungen: (Fortsetzung)
**Decheme, 3 und 4 Oktober 1985, Gentechnik und Molekularbiologie, S. 15-16**

## Beschreibung

Gegenstand der Erfindung ist das Plasmid p SVH 1 und seine Verwendung zur Herstellung eines Hybridvektors, durch den Streptomyceten-DNA auf andere Mikroorganismen, insbesondere auf andere Streptomyceten-Arten übertragen werden kann.

Es ist aus der deutschen Offenlegungsschrift 3 005 226 bekannt, dass aus einer Kultur von Streptomyces espinosus das Plasmid pUC 6 gewonnen werden kann. Es ist weiterhin aus der PCT-Anmeldung 79/01169 bekannt, dass aus Streptomyces lividans ein Plasmid gewonnen werden kann, welches als Vektor für die Einführung von DNA in einige Streptomyces-Arten geeignet erscheint.

Hieraus ist erkennbar, dass der Anwendung gentechnologischer Methoden auf Antibiotika bildende Streptomyces-Arten besondere Bedeutung beigemessen wird. Jedoch sind die meisten bisher bekannten Plasmide für die praktische Anwendung von geringerem Interesse geblieben, da sie sich einmal nicht leicht in grösserer Menge darstellen lassen. Zum anderen ist es für gentechnologische Arbeiten sehr wichtig, Plasmide, die in vielen Kopien pro Zelle vorliegen (= Multicopy-Plasmide), zur Verfügung zu haben, wenn sie als Vektoren für die Amplifizierung klonierter Gene sinnvoll Anwendung finden sollen.

Die Anmelderin hat sich die Aufgabe gestellt, ein Streptomyces-Plasmid zur Verfügung zu stellen, das zur Herstellung von Hybridvektoren geeignet ist, das in hoher Kopienzahl vorliegt, das eine günstige Molekülgrösse hat und zwei singuläre Restriktionstellen hat.

Es hat sich nun gezeigt, dass diese Aufgabe gelöst wird durch das Plasmid p SVH 1, welches aus einer Kultur von Streptomyces venezuelae DSM 40 755 gewonnen wird, ein Molekulargewicht von 8,4 Megadaltons, eine Konturlänge von 4,1 μm aufweist und 12,6 Kilobasen (= kb) umfasst.

Der hier verwendete Stamm von Streptomyces venezuelae ist im einzelnen beschrieben von L. Ettlinger, R. Corbatz und R. Hütter, Arch. Mikrobiol. 31, 326–358 (1958), insbesondere ab Seite 352. Eine weitere Beschreibung findet sich bei R. Hütter «Systematik der Streptomyceten» Karger-Verlag, Basel/New York, 1967, Seite 60.

Streptomyces venezuelae DSM 40755 ist vor allem deshalb als ein vorzüglicher Lieferant von Plasmiden anzusehen, weil hier in jeder Zelle weit mehr als 20 Plasmide vorliegen, welche sich durch ihr niedriges Molekulargewicht von 8,4 Megadaltons besonders gut für die Durchführung von gentechnologischen Arbeiten eignen.

Die Isolierung der Plasmide aus Streptomyces venezuelae DSM 40755 erfolgt nach an sich bekannten Verfahren. Zunächst wird der Stamm in einem geeigneten, glycinhaltigen Medium angezüchtet. Nach dem Ernten, Waschen und Homogenisieren des Myzels werden die Zellwände mit Lysozym entfernt. Nach Behandlung der Zellen mit Proteinase K und Natriumdodecylsulfat lysieren die Zellen, so dass Zellreste sowie chromosomale DNA anschliessend durch Zentrifugation abgetrennt werden können. Danach werden die Plasmide mit Polyethylenglykol gefällt und für anschliessende Schnelluntersuchungen nach einem von H.C. Birnboim und J. Doly, J. Nucl. Acids Res. 7, 1513–1523 (1975) beschriebenen Verfahren aufgearbeitet. Das so angereicherte Plasmid p SVH 1 kann ohne weiteres für analytische Untersuchungen eingesetzt und der Behandlung mit Restriktionsendonukleasen unterworfen werden. Eine präparative Reindarstellung gelingt durch zwei aufeinander folgende Cäsiumchlorid-Dichtegradienten-Zentrifugationen.

Das so gewonnene Plasmid p SVH 1 lässt sich durch endonukleolytische Spaltung mit Restriktionsenzymen auf Agarosegelen charakterisieren. Damit lassen sich Zahl und Grösse der jeweiligen p SVH 1-Fragmente erkennen. Kennzeichnend für das Plasmid pSVH 1 ist, dass es für die Restriktionsendonukleasen Eco R I, Hind III, Xba 1, Hpa 1 keine Schnittstellen besitzt, von den Restriktionsendonukleasen Pst I und Bcl I nur einmal geschnitten wird, von Xho I in zwei Fragmente mit den Längen 7,6 und 4,9 kb, von Cla I in zwei Fragmente mit den Längen 11,8 und 0,8 kb, von Bgl II in drei Fragmente mit den Längen 7,5, 2,8 und 2,4 kb und von Bam H I in vier Fragmente mit den Längen 7,7, 2,0, 1,8 und 1,2 kb zerlegt wird. Die Schnittstellen einiger dieser Enzyme wurden zudem gegeneinander auf dem ringförmigen Plasmidmolekül vermessen (siehe beiliegende Figur). Ausserdem entstehen durch endonukleolytische Spaltung mit Pvu II und Kpn I 4, mit Nru I, Pvu I und Sma I 5, mit Sst II und Sac II 7, mit Sst I 8 und mit Sal I weit mehr als 10 Fragmente.

Konturmessungen an einer Vielzahl von Plasmiden ergaben eine Grösse von 4,1 μm bezw. ein daraus abgeleitetes Molekulargewicht von 8,23 Megadaltons. Dieser Wert steht in guter Übereinstimmung mit Molekulargewichten, die aus der Addition von Restriktionsfragmentgewichten nach gelelektrophoretischer Auftrennung berechnet wurden (= 8,4 Megadaltons). Identische oder ähnliche Plasmide sind bisher in keiner anderen Streptomyces venezuelae Biotype gefunden worden [vgl. V.S. Malik und F. Reuser, Plasmid 2, 627–31 (1979)].

Das Plasmid p SVH 1 ist zur Herstellung eines Vektors aus mehreren Gründen sehr geeignet. Vor allem ist die ausserordentlich hohe Kopienzahl von weit mehr als 20 Plasmiden pro Zelle und seine Stabilität eine ganz wichtige Voraussetzung für den Einsatz von p SVH 1 für gentechnologische Arbeiten. Bisher wurden plasmidfreie Stämme bei Streptomyces venezuelae DSM 40755 trotz umfangreicher Untersuchungen nicht beobachtet. Bei Plasmidisolierungen wurden stets Ausbeuten von mehr als 200 μg, berechnet auf einen Liter ursprünglicher Streptomycetenkultur, erzielt.

Das Plasmid p SVH 1 eignet sich vor allem zur Bildung eines Hybridvektors, der in andere Streptomyceten-Arten eingeführt werden kann. Es hat sich nämlich gezeigt, dass alle bisher bekannten Plasmide sich nur in relativ wenigen Wirtszellen etablieren lassen. So ist z.B. bekannt, dass zwi-

schen grampositiven und gramnegativen Bakterien Barrieren für eine Vermehrung fremder DNA bestehen (vgl. P. Courvalin und M. Fiandt, Gene 8, 247–269 (1980)). Die grössten Chancen zu einer erfolgreichen Klonierung bestehen immer dann, wenn der Hybridvektor in eine nahe verwandte Wirtszelle eingebracht wird. Alle bisher publizierten Daten über Streptomycetenplasmide und -phagen deuten sogar darauf hin, dass diese nur in eine sehr limitierte Zahl anderer Streptomyceten stabil eingebracht werden können. Eine Klonierung von Streptomyceten-DNA in Streptomyceten wird immer dann erforderlich werden, wenn es sich nicht nur um eine oder wenige zu klonierende Gene handelt, sondern um eine ganze Reihe von Genen, die etwa an der Synthese eines Antibiotikums beteiligt sind.

Eine Klonierung der Gene für einen ganzen Stoffwechselweg stellt zumindest eine gewaltige Arbeitsaufgabe dar, wenn sie nicht sogar – bedingt durch eine etwaige Streuung der Gene über das ganze Streptomycetengenom – zu einem praktisch unlösbaren Problem wird. Bei einem Streptomyceten-Klonierungssystem kann hier, z.B. wenn ein Stoffwechselengpass vorliegt, schon u.U. die Amplifikation eines einzelnen Gens genügen, um wesentlich höhere Ausbeuten zu erzielen. Auch bei der Schaffung hybrider Antibiotika, wie sie durch Kombination eng verwandter Stoffwechselwege – z.B. durch Einbringen eines ein Antibiotikum modifizierenden Enzyms – denkbar ist, ist aus denselben Gründen nur ein Streptomyceten-Wirtsvektor-System erfolgversprechend.

Zur Herstellung des Hybridvektors werden dabei die gleichen gentechnologischen Methoden angewendet, die bei Escherichiacoli-Plasmiden bereits früher eingesetzt wurden und auch bei Streptomyceten von M.Bibb, J.C. Schottel, S.N. Cohen, Nature 284, 526–531 (1980), und C.J. Thompson, J.M. Ward, D.A. Hopwood, Nature 286 525–527 (1980), angewendet wurden.

Nach diesen bekannten Verfahren können in das Plasmid p SVH 1 in einige der mit den oben genannten Restriktionsendonukleasen erhaltenen Schnittstellen nicht nur Gene, die Antibiotika-Resistenzen tragen, eingefügt werden, sondern auch Gene, die eine Steigerung der Antibiotika-Produktion bewirken. Die so erhaltenen Hybrid-Plasmide sind nach allen bisher vorliegenden Beobachtungen in Streptomyces-Zellen ebenso lebens- und vermehrungsfähig wie das Ausgangsplasmid.

Die Erfindung wird durch das folgende Beispiel weiter veranschaulicht, in dem sich Prozentangaben auf das Gewicht beziehen, sofern nichts anderes angegeben ist.

Beispiel

1. Anzucht und Protoplastierung von Streptomyces venezuelae DSM 40755

Die Anzucht erfolgt in geeigneten Gefässen, z.B. 300 ml Erlenmeyerkolben in 50 ml Medium, z.B. Luria Broth oder 2 YT (vgl. J.H. Miller, Experiments in Molecular Genetics, Cold Spring Harbor Laboratory, 1972) oder in anderen Medien mit verwertbarer Stickstoff- und Kohlenstoff-Quelle. Alle Medien enthalten zusätzlich 0,5 Gew.-% Glycin. Angeimpft wird entweder mit einer Sporensuspension oder einer Öse voller Sporen. Die Induktion erfolgt bei 32 °C in einer Schüttelmaschine, Auslenkung 4 cm, bei 220 Umdrehungen/min für 2–3 Tage bis zur frühen stationären Phase. Anschliessend werden die Zellen durch eine 10minütige Zentrifugation bei 6000 g und 4 °C geerntet. Für die Protoplastierung zur anschliessenden Transformation mit Plasmiden wurde dieser und die folgenden Schritte unter sterilen Bedingungen vorgenommen. Das Mycelpellet wird einmal mit Wasser gewaschen und dann in 5 ml geeignetem hypertonischen Puffer, beispielsweise bestehend aus 25% Saccharose, 50 mM Ethylendiamintetraessigsäure (= EDTA) und 50 mM Tris(hydroxymethylamino)methan(Tris) bei einem pH 8, aufgenommen. Anschliessend wird das Mycel durch zwei bis vier Schläge im Glashomogenisator aufgebrochen. Hierdurch wird dem Lysozym der Zugang zu seinem Wirkort erleichtert. Eine Verdauung nach Zufügung von 1 ml Lysozym (enthaltend 10 mg/ml in 50 mM Tris bei einem pH 8) bei Zimmertemperatur für 60 Minuten ist ausreichend, um eine praktisch vollständige Protoplastierung zu erreichen.

2. Lyse der Zellen

Für die Präparation von Plasmid-DNA werden die Protoplasten weiter eine halbe Stunde bei Zimmertemperatur mit einem Milliliter Proteinase K (enthaltend 1 mg/ml in 50 mM Tris bei einem pH 8) behandelt. Anschliessend werden die Zellsuspensionen mit Natriumdodecylsulfat bis zu einer Endkonzentration von 0,1% versetzt und dann eine weitere halbe Stunde bei Zimmertemperatur inkubiert. Anschliessend werden 7 ml einer Lösung von 2 mM EDTA, 2 M Natriumchlorid, 50 mM Tris bei einem pH 8 zugegeben und die hochviskose Mischung mindestens eine Stunde auf Eis inkubiert. Zellreste sowie die überwiegende Menge der chromosomalen DNA werden anschliessend durch einstündige Zentrifugation bei 20 000 g und 4 °C abzentrifugiert und unter verschiedenen Bedingungen aufgearbeitet, je nachdem, ob eine analytische oder präparative Plasmid-Isolierung angestrebt wird.

3. a) Analytische Plasmid-Isolierung

Der Überstand der Zentrifugation wird vorsichtig abgegossen und die Plasmid-DNA durch Zufügen von Polyethylenglykol 6000 bis zu einer Endkonzentration von 10% ausgefällt. Nach einer einstündigen Inkubation auf Eis wird die Fällung durch eine 20 Minuten dauernde Zentrifugation bei 20 000 g und 4 °C gesammelt, das Pellet in 500 µl 0,2 N NaOH aufgenommen und eine halbe Stunde bei 0 °C inkubiert. Anschliessend wird die alkalische Suspension durch Zufügen von 375 µl 3 molarer Natriumacetatlösung von einem pH 4,8 neutralisiert. Dadurch wird die durch die Alkalibehandlung teilweise in Einzelstränge denaturierte chromosomale DNA in Regionen partieller Homologie wieder in doppelsträngige DNA überführt und es entsteht durch Quervernetzung der chromosomalen DNA-Fragmente ein Produkt eines riesigen Molekulargewichts, das sich leicht durch 5minütige Zentrifugation bei 15 000 g und 4 °C entfernen lässt [vgl. H.C. Birnboim und J. Doly, Nucleic Acids Research 7, 1513–23 (1979)]. Der Überstand wird anschliessend mit dem 2,5fachen Volumen Ethanol versetzt und nach Inkubation bei −18 °C für 4 Stunden wird die gefällte Plasmid-DNA durch Zentrifugation (15 000 g, 4 °C, 20 Minuten) gesammelt und einmal mit 78%igem Ethanol gewaschen. Die angereicherte Plasmid-DNA kann dann in 0,1 mM EDTA, 20 mM Tris bei einem pH 8 aufgenommen und für die rasche Analyse des Plasmids mit Restriktionsenzymen verwendet werden.

b) Präparative Plasmid-Isolierung

Nach der bereits bei der analytischen Plasmid-Isolierung beschriebenen Polyethylenglykolfällung wird die durch eine 20minütige Zentrifugation bei 20 000 g und 4 °C gesammelte Plasmid-DNA in 6 ml 10 mM EDTA, 50 mM Tris bei einem pH 8 aufgenommen. Das Volumen der entstandenen Suspension wird mit einer Pipette gemessen und pro Milliliter Suspension 1 g Caesiumchlorid zugefügt. Anschliessend werden 200 μl Ethidiumbromid (10 mg/ml) zugefügt und die Suspension bei 80 000 g und 15 °C für 60 Stunden in der Ultrazentrifuge zentrifugiert. Die dichtere Bande wird sichtbar gemacht durch UV-Licht, durch seitliches Punktieren mit einer Injektionsnadel abgezogen und erneut mit 5 ml einer Lösung aus 10 mM EDTA, 50 mM Tris und einem pH 8 versetzt, die zusätzlich noch 5 g Caesiumchlorid enthält. Die Rezentrifugation in der Ultrazentrifuge erfolgt unter den gleichen Bedingungen wie auch die anschliessende Isolierung der Bande. Die gesammelte DNA wird dann mehrfach zur Entfernung von Ethidiumbromid mit Caesiumchlorid-gesättigtem n-Butanol extrahiert und die wässrige Phase gegen 10 mM EDTA, 50 mM Tris bei einem pH 8 mindestens 6 Stunden dialysiert. Das Dialysat wird anschliessend mit dem 2½fachen Volumen Ethanol versetzt und nach 4 Stunden bei −18 °C kann ausgefallene Plasmid-DNA durch 20minütige Zentrifugation bei 15 000 und 4 °C pelletiert werden. Der Niederschlag wird anschliessend einmal mit 78%igem Ethanol gewaschen und in 0,1 mM EDTA, 20 mM Tris bei einem pH 8 und 4 °C aufbewahrt.

4. Charakterisierung der Plasmid-DNA

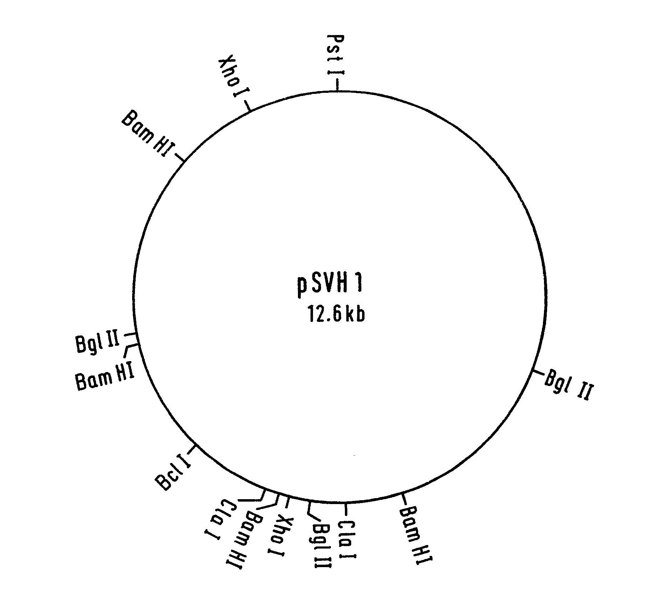

Die elektronenmikroskopische Untersuchung erfolgte nach Standard-Verfahren [A.K. Kleinschmidt, Monolayer Techniques, in Electron Microscopy of Nucleic Acid Molecules, in Methods of Enzymology, S.P. Colowick und N.O. Kaplan, Verlag Academic Press, Band 25, 361–377 (1968)]. Danach wurde die Konturlänge mit 4,1 μm bestimmt. Aus der Konturlänge des Plasmids konnte ein Molekulargewicht von 8,4 Megadaltons bestimmt werden. Die Charakterisierung des Plasmids p SVH 1 wurde mit einer grossen Zahl verschiedener Restriktionsenzyme durchgeführt. Die verdaute DNA wurde anschliessend auf 0,7%igen, horizontalen Agarosegelen (2 mM EDTA, 40 mM Natriumacetat, 80 mM Tris, pH 8,3) in einem Voltgradienten von 5 V/cm für 4 Stunden elektrophoretisch aufgetrennt. Anhand von mitelektrophorierten Markerfragmenten bekannter Grösse kann zudem durch einen Vergleich der Wanderungsstrecken im elektrischen Feld das Molekulargewicht der unbekannten Fragmente bestimmt werden. Die Schnittstellen von 6 Restriktionsenzymen wurden genau auf dem ringförmigen p SVH 1-Molekül gegeneinander vermessen (vgl. die beiliegende Figur).

Der einzige Pst I-Schnittstelle auf dem Plasmid wurde dabei willkürlich als Nullpunkt gesetzt. Die Lage der Schnittstelle eines Enzyms, z.B. von Bam H I, auf Fragmenten, wie sie durch die Behandlung mit einer anderen Restriktionsendonuklease entstehen, wurde durch zwei sukzessive Verdauungen bestimmt. Nach Restriktion mit dem Enzym Bam H I wird ein Aliquot direkt auf das Gel aufgetragen, ein anderes Aliquot wird mit Phenol, das mit 100 mM Tris-Puffer von einem pH 8 gesättigt wurde, zweimal extrahiert und die wässrige Phase zur Entfernung des Phenols dreimal mit Ether extrahiert. Anschliessend wird die DNA mit dem 2½fachen Volumen Ethanol gefällt und kann dann, im Verdauungspuffer suspendiert, der Einwirkung des zweiten Enzyms ausgesetzt werden. Die doppelt verdaute DNA wird nun ebenfalls wie auch eine Plasmidprobe, die lediglich mit dem zweiten Enzym behandelt worden ist, auf das Gel aufgetragen. Auf diese Weise kann das Fragment bestimmt werden, auf dem das zweite Enzym eine Restriktionsstelle besitzt.

Die Anzahl der Kopien von p SVH 1 mit wesentlich mehr als 20 Kopien pro Zelle wurde auf folgende Weise bestimmt: Den Zellen von Streptomyces venezuelae DSM 40755 wurden in der Schüttelkultur Kohlenstoff-14-markierte Thymidin-Moleküle angeboten, die in der Zelle gleichmässig in die chromosomale und in die Plasmid-DNA eingebaut werden. Nach Lyse der Zellen und Auftrennung der Rohlysate ohne vorherige Abtrennung der chromosomalen DNA mit Hilfe des Caesiumchlorid-Dichtegradienten in Gegenwart von Ethidiumbromid wird der Gradient anschliessend in etwa 50 Fraktionen zerlegt und die Radioaktivität der einzelnen Fraktionen bestimmt. Aus dem Verhältnis der Aktivität der dichteren Plasmid- und der leichteren chromosomalen Bande kann bei Kenntnis der Molekulargewichte des Plasmids und des abgeschätzten Molekulargewichts des Chromosoms die Kopienzahl des Plasmids pro Zelle berechnet werden [vgl. R. Radloff, W. Bauer und J. Vinograd, Proc. Nat. Acad. Sci. U.S., 57, 1514–1520 (1967)].

Transformation von Streptomyceten mit dem Plasmid p SVH 1

Es wurde das von Hopwood und Mitarbeitern entwickelte Transformationssystem verwendet [M.J. Bibb, J.M. Ward und D.A. Hopwood, Nature 274, 398–400 (1978)]. Die Protoplasten werden wie unter (1.) beschrieben hergestellt und abzentrifugiert (6000 g, 4 °C, 5 Minuten) und in einem Protoplasten-Medium (25% Sucrose, 1,5 mM $K_2SO_4$, 10 mM $MgCl_2$, 0,36 mM $KH_2PO_4$, 25 mM $CaCl_2$, 25 mM NaCl, 25 mM Tris, pH 7,2) aufgenommen.

Nach 30minütiger Inkubation bei 4 °C werden die Protoplasten erneut pelletiert und das Pellet in 1 ml frischem Protoplastenmedium durch vorsichtiges Auf- und Abpipettieren suspendiert. Anschliessend wird das Plasmid p SVH 1 zugefügt und die Suspension mit 3 ml 30%igem Polyethylenglykol 1000 versetzt, gemischt und weitere 4 Minuten bei 0 °C inkubiert. Dann werden 8 ml Protoplastenmedium zugefügt und die erhaltene Suspension wiederum abzentrifugiert. Das Pellet wird anschliessend mit einem modifizierten Regenerationsmedium (25% Sucrose, 30% Glucose, 1,5 mM $K_2SO_4$, 10 mM $MgCl_2$, 0,1% Casaminosäuren, 0,5% Hefeextrakt, 0,36 mM $KH_2PO_4$, 20 mM $CaCl_2$, 0,3 mM L-Prolin, 25 mM NaCl, 25 mM Tris, pH 7,2) überschichtet und 2 Stunden im Schüttelwasserbad bei 37 °C inkubiert. Die entstandene Zellsuspension wird dann zur Isolierung von Einzelkolonnien auf geeignete selektive Medien enthaltende Agarplatten ausplattiert und Transformanten können mit dem unter 3 (a) beschriebenen Schnell-Löseverfahren analysiert und die darin enthaltene Plasmid-DNA mit Restriktionsenzymen charakterisiert werden.

6. Klonierung fremder DNA in dem Plasmid p SVH 1

Das Plasmid p SVH 1 wird mit einem Restriktionsenzym geöffnet. Gleichzeitig wird die in das Plasmid einzubauende chromosomale DNA so geschnitten, dass Schnittstellen mit gleichen, d.h. miteinander ligierbare, Enden entstehen. Nach 3minütigem Erhitzen auf 70 °C werden beide Ansätze gemischt, mit Phenol extrahiert und mit Ethanol gefällt wie unter (4.) beschrieben. Anschliessend wird die DNA im Ligasepuffer (nach Massgabe der Hersteller) aufgenommen, mit T4-DNA-Ligase versetzt und bei 16 °C eine Stunde und unter allmählicher Abkühlung auf 4 °C über Nacht inkubiert. Das so ligierte Plasmid wird dann, wie unter (5.) beschrieben, anschliessend in einen geeigneten Streptomyceten eingeführt.

**Patentansprüche**
für die Vertragsstaaten BE, CH, DE, FR, GB, IT, LI, NL, SE:

1. Plasmid p SVH 1, erhältlich aus Streptomyces venezuelae DSM 40 755, gekennzeichnet durch ein Molgewicht von 8,4 Megadalton.
2. Verwendung des Plasmids p SVH 1 gemäss Anspruch 1 zur Herstellung eines Hybridvektors.

**Patentansprüche**
für den Vertragsstaat AT

1. Verfahren zur Herstellung des Plasmids p SVH 1 mit einem Molekulargewicht von 8,4 Megadalton dadurch gekennzeichnet, dass es aus einer Kultur von Streptomyces venezuelae DSM 40 755 isoliert wird.
2. Verwendung des nach Anspruch 1 erhaltenen Plasmids p SVH 1 zur Konstruktion eines Vektors.

**Claims**
for the Contracting states BE, CH, DE, FR, GB, IT, LI, NL, SE

1. Plasmid p SVH 1, which is obtainable from Streptomyces venezuelae DSM 40 755, char-acterized by a molecular weight of 8.4 megadaltons.
2. The use of the plasmid p SVH 1 according to claim 1 for the production of a hybrid vector.

**Claims**
for the Contracting state AT:

1. A process for the preparation of the plasmid p SVH 1 having a molecular weight of 8.4 megadaltons, characterized by isolating it from a culture of Streptomyces venezuelae DSM 40 755.
2. Use of the plasmid p SVH 1 obtained according to claim 1 for the construction of a vector.

**Revendications**
pour les Etats contractants BE, CH, DE, FR, GB, IT, LI, NL, SE

1. Plasmide p SVH 1, obtenable à partir de Streptomyces venezuelae DSM 40 755, caracté-risé par une masse moléculaire de 8,4 mégadal-tons.
2. Utilisation du plasmide p SVH 1 selon la revendication 1, pour la préparation d'un vecteur hybride.

**Revendications**
L'Etat contractant AT

1. Procédé de préparation du plasmide p SVH 1 ayant une masse moléculaire de 8,4 mégadal-tons, caractérisé en ce qu'il est isolé à partir d'une culture de streptomyces venezuelae DSM 40 755.
2. Utilisation du plasmide p VSH 1 obtenu selon la revendication 1 pour la construction d'un vec-teur.

pSVH 1
12.6 kb